# EUROPEAN PATENT APPLICATION

(11) **EP 0 776 667 A2**
(43) Date of publication of application: **04.06.1997**
(21) Application number: 96106838.4
(22) Date of filing: 30.04.1996
(51) Int. Cl.: A61K 35/78, A61K 31/045

(54) **Anti-ulcerative drug**

(30) Priority: 30.11.1995 JP 336104/95
(71) Applicant: NIPPON MEKTRON, LTD., Minato-ku Tokyo (JP)
(72) Inventor: Nakamura, Motoyuki, Kitaibaraki city, Ibaraki (JP); Nakasumi, Tetsuo, Cidade Sao Paulo, Estado Sao Paulo (BR); Yoshizawa, Toyokichi, Kitaibaraki city, Ibaraki (JP); Minagawa, Yumiko, Kitaibaraki city, Ibaraki (JP); Nakagawa, Keiji, Takahagi city, Ibaraki (JP)
(74) Representative: Hansen, Bernd, Dr. Dipl.-Chem.

(57) **Abstract**

Methanol extract of Maytenus ilicifolia, powder obtained as an aqueous layer component and oily substance obtained a chloroform layer component by intersolvent distribution of a methanol extract between chloroform and water, chromatographic eluate fractions by polystyrene-based resin of the powder obtained as the aqueous layer component, n-hexane insoluble substance obtained from the oily substance as the chloroform layer component, and friedelan-3-β-ol contained therein have a distinguished anti-ulcerative effect on gastric mucosa lesions caused by ethanol.

## Description

### BACKGROUND OF THE INVENTION

### 1. FIELD OF THE INVENTION

The present invention relates to an anti-ulcerative drug, and more particularly to an anti-ulcerative drug comprising an extract from plants as an effective component.

### 2. RELATED PRIOR ART

So many compounds having an anti-ulcerative effect have been so far reported, and most of them are synthesized by chemical means, and it is inevitable avoid various side effects, when administered continuously, due to their nature.

### SUMMARY OF THE INVENTION

An object of the present invention is to provide an anti-ulcerative drug comprising an extract from plants having no such side effects as an effective component.

According to the present invention, there is provided an anti-ulcerative drug comprising an alcohol extract of Maytenus ilicifolia as an effective component.

### DETAILED DESCRIPTION OF THE INVENTION

Maytenus ilicifolia is a plant of the family Celastraceae used as a folk medicine in Brazil and known as a specific remedy for gastric ulcer, duodenal ulcer, intestinal fermentation, etc. In the present invention, its leaves are used, but it has not been known at all that its extract has a remarkable anti-ulcerative effect.

Alcohol extraction is carried out as follows:
Raw leaves, or dried and pulverized leaves of Maytenus ilicifolia are extracted with an alcohol such as methanol, ethanol, n-butanol, etc., preferably methanol, usually under reflux condition and then alcohol is distilled off from the extract to obtain a dark green powdery alcohol extract.

The thus obtained alcohol extract is subjected to intersolvent distribution between chloroform and water (purified water) and the resulting aqueous layer component is concentrated under reduced pressure, whereby yellowish brown powders can be obtained. The yellowish brown powders are apllied to various chromatographies and successive elutions with water, alcohol or ketone of various concentrations, whereby fractions having a high anti-ulcerative effect can be obtained. More specificelly, the yellowish brown powders obtained from the aqueous layer component are dissolved into water and the resulting aqueous solution is apllied to a column chromatography using polystyrene-based resin and elute with water and alcohol or ketone of various concentrations. The resulting eluates as well as the aqueous layer before the fractionation have been found to show a distinguished anti-ulcerative effect. When the chloroform layer component is concentrated, dark green oily substance are obtained and has been found to show a distinguished anti-ulcerative effect as well.

When the oily substance obtained from the chloroform layer compnent is dissolved into n-hexane and the solvent, i.e. n-hexane, is distilled off from the resulting solution, dark green oily substance can be obtained, whereas black oily substance can be obtained from the insoluble residue.

When the n-hexane-insoluble black oily substance are applied to silicagel chromatography and elute with chloroform to conduct fractionation into fractions Nos. 1 to 100, each in 20ml, friedelan-3-on (friedelin) can be obtained from fractions Nos. 10 to 15, and friedelan-3-β-ol (epifriedelinol) can be obtained from fractions Nos. 24 to 50. These compounds are known compounds and identification of friedelan-3-β-ol having an anti-ulcerative effect has been conducted by comparison of its physico-chemical properties. The friedelan-3-β-ol has been found to show anti-ulcerative effect on the gastric mucosa lesions caused by ethanol.

These extracts and fractionates can be provided in the form of powder, granules, tablets, sugar coated tablets, capsules, liquid preparations or the like and can be administered perorally, parenterally, by inhalation, by perrectum, locally or the like. The parenteral administration includes a subcutaneous injection, an intravenous injection, an intramuscular injection, an intranasal administration or injection. A dose is usually in a range of about 0.1 to about 500 mg/kg body weight, and an exact dose is determined within the above-mentioned range, depending on ages, body weights and symptoms of patients and also administration route, etc. and administration is made usually one to five times a day. Their toxicity is low and investigation of acute toxicity on male Wistar rats by oral administration revealed that no killing occurred even at 3,000 mg/kg (p.o.)

Alcohol extracts of Maytenus ilicifolia and friedelan-3-β-ol as its one component have a remarkable anti-ulcerative effect, particularly a distinguished ameliorating effect on the gastric mucosa lesions caused by hydrochloric acid-ethanol to the acute gastric ulcer model, and thus it seems that these extracts have a protective factor-intensifying action.

Furthermore, friedelan-3-β-ol has been also found to show an analgesic-anti-inflammatory effect at the same time, though it is somewhat less effective in the anti-ulcerative action than the commercially available anti-ulcerative drugs. Thus, this compound can be used as an - analgesic-anti-inflammatory drug as well as an anti-ulcerative drug without any side effects such as gastrointestinal disorder (generation of ulcer), as caused by non-steroid anti-inflammatory analgesics.

### BRIEF DESCRIPTION OF THE DRAWINGS

Fig.1 is a graph showing an anti-ulcerative effect when Samples 2 to 7 are administered at a dose of 250 mg/kg, respectively.

Fig.2 is a graph showing an anti-ulcerative effect when Samples 2 to 7 are administered at a dose of 125 mg/kg, respectively.

Fig.3 is a graph showing an anti-ulcerative effect when Sample 11 is administered at a dose of 125 mg/kg.

### PREFERRED EMBODIMENTS OF THE INVENTION

The present invention will be described in detail below, referring to Example.

### EXAMPLE

3.2kg of dried crude Maytenus ilicifolia was twice extracted with 30 liters of methanol with heating at the reflux temperature, and then the solvent was distilled off, whereby 153.3g of methanol extract (Sample 1) was obtained. The methanol extract was subjected to intersolvent distribution between chloroform and water, and the respective solvents were distilled off, whereby 58.0g of dark green oily substance (Sample 2) was obtained from the chloroform layer component and 82.9g of yellowish brown powders (Sample 3) was obtained from the aqueous layer component.

(1) Sample 3 obtained from the aqueous layer component was dissolved in 300ml of water and the resulting solution was applied to chromatography using 1,000ml of polystyrene-based resin (styrene-divinylbenzene-based copolymer resin, Diaion HP-20, trademark of a product made by Mitsubishi Kagaku K. K., Japan) and to fractionation into the following 4 eluate fractions, each in 1,000ml.

| | |
|---|---|
| Sample 4: H₂O eluate fraction | 58.8g |
| Sample 5: 50% MeOH eluate fraction | 8.7g |
| Sample 6: 100% MeOH eluate fraction | 4.5g |
| Sample 7: 50% Me₂CO eluate fraction | 10mg |

Effect on gastric mucosa lesions caused by ethanol:
Suspensions each containing such amounts of these extracts and fractionation samples in aqueous 0.5% sodium carboxymethylcellulose (CMC) solution, respectively, as to give dose of 250 mg/kg or 125 mg/kg were prepared and administered perorally by force into male CrJ Wister rats, which had been subjected to quarantine acclimation for one week after purchase at the age of 6 weeks, through a metallic cathether at a dose of 0.5 ml/rat, thereby carrying out a pretreatment for administration of an ulcer-inducing agent. The same volume of the aqueous CMC solution was administered to the lesion model group, whereas Cetraxate as a commercially available anti-ulcerative drug was administered at a dose of 180 mg/kg to the control drug group.

Then, the rats were kept away from feeding of food and water for 24 hours and then 1.5ml of hydrochloric acid-ethanol (150mM hydrochloric acid + 60% ethanol) was perorally administered. One hour thereafter, the rats were left under etheral anesthesia to kill, and stomachs were taken out therefrom and simply fixed in 3% formalin. Then, the stomachs were incised along the greater curvature to visually observe the occurrences of ulcers and also to take pictures and conduct computer image processing of the pictures to determine percent ulcer generation (percentage of generated ulcer area to total stomach inner surface area). Test drugs were perorally administered at the time of one hour before the administration of hydrochloric acid-ethanol.

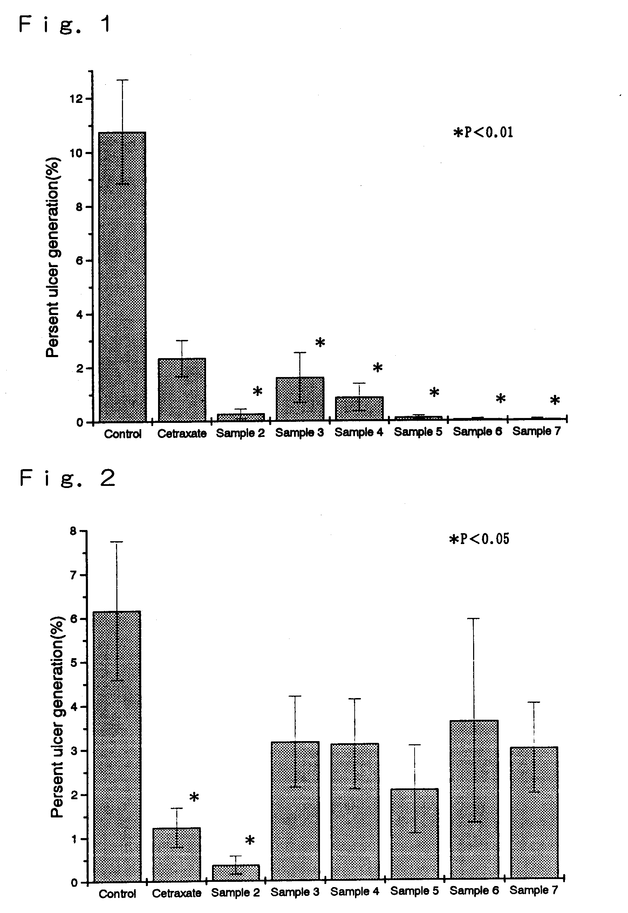

Results as shown in the following Table 1 and Figs. 1 and 2 were obtained. It can be seen therefrom that at a dose of 250 mg/kg (P<0.01) control effect was obtained on all the Samples-pretreated groups and at a dose of 125 mg/kg (P<0.25) a significant control effect was obtained in the case of chloroform layer (dark green oily substance), i.e. Sample 2.

**TABLE 1**

| | Percenr ulcer generation (average ± standard deviation, %) | |
|---|---|---|
| | 250 mg/kg | 125 mg/kg |
| Control | 10.76±1.91 | 6.17±1.58 |
| Cetraxate | 2.35±0.67 | 1.24±0.45 |
| Sample 2 | 0.27±0.18 | 0.37±0.21 |
| Sample 3 | 1.61±0.92 | 3.17±1.03 |
| Sample 4 | 0.87±0.51 | 3.11±1.02 |
| Sample 5 | 0.13±0.06 | 2.07±1.00 |
| Sample 6 | 0.06±0.03 | 3.62±2.31 |
| Sample 7 | 0.05±0.03 | 3.00±1.02 |
| (Notes) Control : aqueous 0.5% CMC solution Cetraxate: 180 mg/kg | | |

Still furthermore, said Sample 2 was dissolved in n-hexane, and the solvent was distilled off from the solution, whereby 12.0g of dark green oily substance (Sample 8) was obtained, whereas 41.9g of black oily substance (Sample 9) was obtained from the undissolved part.

11g of this Sample 9 was subjected to chromatography using 100ml of silica gel column (Kieselgel Art 7734, trademark of a product made by Merck & Co., Inc.) and elute with chloroform to fractionate the eluate into fractions Nos.1 to 100, each in 20ml.

116mg of light yellow crystal was obtained from fractions Nos. 10 to 15 and recrystallized from chloroform, whereby 90mg of colorless needle-like crystal (Sample 10) was obtained.
- ¹³C-NMR(CDCl₃-d)δ:: 213.2(s), 59.5(d), 58.2(d), 53.1(d), 42.8(d), 42.1(s), 41.5(t), 41.3(t), 39.7(s), 39.2(t), 38.3(s), 37.4(s), 36.0(t), 35.6(t), 35.3(t), 35.0(q), 32.7(t), 32.4(t), 32.1(q), 31.8(q), 30.5(t), 30.0(s), 28.2(s), 22.3(t), 20.2(q), 18.7(q), 18.2(t), 17.9(q), 14.6(q), 6.8(q)

The foregoing physico-chemical properties were found to be identical with those of friedelan-3-on (friedelin) represented by the following chemical formula, as disclosed in Magn. Reson. Chem. 23, page 616 (1985) and 25, page 39 (1987).

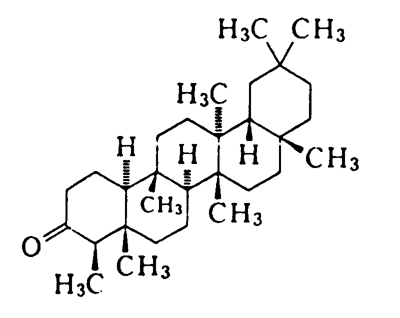

Furthermore, 550mg of light yellowish green powder was obtained from fractions Nos. 24 to 50, and recrystallized from chloroform, whereby 470mg of colorless crystal (Sample 11) was obtained.
[α]_{D}21.4° (c=0.33, CHCl₃)
HR-MS: m/z 428.4024[M]
- ¹³C-NMR(CDCl₃-d)δ:: 72.8(d), 61.4(d), 53.2(d), 49.2(d), 42.9(d), 41.8(t), 39.7(s), 39.3(t), 38.4(s), 37.9(s), 37.1(s), 36.1(t), 35.6(t), 35.4(t), 35.2(t), 35.0(q), 32.8(t), 32.4(t), 32.1(q), 31.8(q), 30.7(t), 30.0(s), 28.2(s), 20.1(q), 18.7(q), 18.3(q), 17.6(t), 16.4(q), 15.8(t), 11.6(q)

The foregoing physico-chemical properties were found to be identical with those of friedelan-3-β-ol (epifriedelinol) represented by the following chemical formula, as disclosed in QUIM ICA NOVA, 31, No.4, page 254 (1990) and J. Am. Chem. Soc., 78, page 5041 (1956).

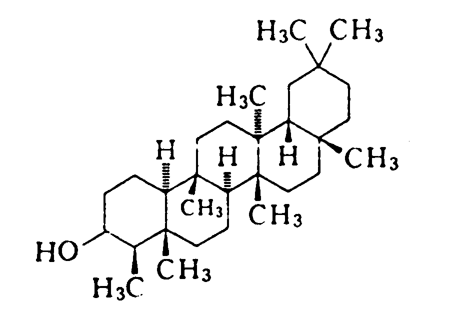

Effect on gastric muscosal lesions caused by ethanol:
Suspension containing such an amount of Ssample 11 in aqueous 0.5% sodium carboxymethylcellulose (CMC) solution as to give a dose of 125 mg/kg was prepared and administered perorally by force into male CrJ Wister rats, which had been subjected to quarantine acclimation for one week after purchase at the age of 6 weeks, through a metallic cathether at a dose of 0.5 ml/rat, thereby carrying out a pretreatment for administration of an ulcer-inducing agent. The same volume of the aqueous CMC solution was administered to the lesion model group, whereas Cetraxate as a commercially available anti-ulcerative drug was administered at a dose of 125 mg/kg to the control drug group. Then, the same successive procedures as mentioned above was conducted.

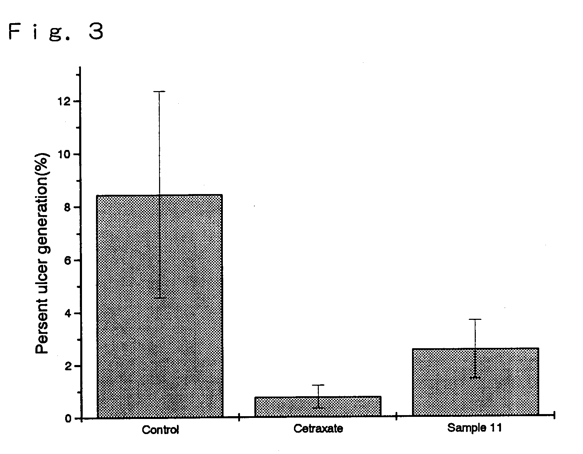

Results (n=5) as shown in the following Table 2 and Fig. 3 were obtained.

**TABLE 2**

| | Percenr ulcer generation (average ± standard deviation, %) |
|---|---|
| Control | 8.46±3.89 |
| Cetraxate | 0.77±0.44 |
| Sample 11 | 2.57±1.11 |

## Claims

1. An anti-ulcerative drug which comprises an alcohol extract of Maytenus ilicifolia.

2. An anti-ulcerative drug according to Claim 1, wherein the alcohol extract is an extract of leaves of Maytenus ilicifolia.

3. An anti-ulcerative drug according to Claim 1, wherein the alcohol extract is a methanol extract.

4. An anti-ulcerative drug which comprises powder obtained as as aqueous layer component by intersolvent distribution of a methanol extract of Maytenus ilicifolia between chloroform and water, as an effective component.

5. An anti-ulcerative drug which comprises a chromatographic eluate fraction by polystyrene-based resin from powder obtained as an aqueous layer component by intersolvent distribution of a methanol extract of Maytenus ilicifolia between chloroform and water, as an effective component.

6. An anti-ulcerative drug which comprises oily substance obtained as a chloroform layer component by intersolvent distribution of a methanol extract of Maytenus ilicifolia between chloroform and water, as an effective component.

7. An anti-ulcerative drug which comprises n-hexane insoluble oil substance obtained as a chloroform layer component by intersolvent distribution of a methanol extract of Maytenus ilicifolia between chloroform and water, as an effective component.

8. An anti-ulcerative drug which comprises friedelan-3-β-ol as an effective component.

9. An anti-ulcerative drug according to Claim 1, wherein the anti-ulcerative effect is on gastric mucosa lesions caused by ethanol.
